Europäisches Patentamt

European Patent Office

Office européen des brevets

(11) Numéro de publication : **0 457 631 A1**

(12) # DEMANDE DE BREVET EUROPEEN

(21) Numéro de dépôt : **91400943.6**

(51) Int. Cl.⁵ : **A61B 5/14**

(22) Date de dépôt : **09.04.91**

(30) Priorité : **10.04.90 FR 9004569**

(43) Date de publication de la demande :
**21.11.91 Bulletin 91/47**

(84) Etats contractants désignés :
**AT BE CH DE DK ES FR GB GR IT LI LU NL SE**

(71) Demandeur : **SOREM**
**Z.A. Lavoisier, Impasse de la Fosse Rouge**
**F-77220 Presles-en-Brie (FR)**

(72) Inventeur : **Collin, Joel-Bernard**
**1, Place des Magnolias**
**F-77680 Roissy-en-Brie (FR)**

(74) Mandataire : **Pinguet, André**
**Cabinet de Propriété Industrielle CAPRI 28**
**bis, avenue Mozart**
**F-75016 Paris (FR)**

(54) **Dispositif permettant le transfert du sang artériel ou veineux d'un système type vacutainer à une seringue.**

(57)  L'invention concerne un dispositif permettant de transférer du sang artériel ou veineux dans une seringue sans risque de contamination par le sang ni par piqûres.

Ce dispositif est constitué d'un corps cylindrique 1 évidé en son milieu de part en part 2 en matière plastique ou caoutchouc, servant de piston au vacutainer.

L'une des extrémités sera de type cône Luer 3 Lock femelle 4 s'adaptant parfaitement sur une seringue 9 à embout Luer Lock mâle 11. L'autre extrémité est composée dans la partie centrale d'une membrane en latex 5 qui doit être centrée par rapport au cône Luer Lock 11 du corps de la seringue.

Lorsque l'utilisateur enfonce le piston 1 vers la protection de l'aiguille 2 du vacutainer 10 sous la pression, la protection de l'aiguille 2 sera rompue libérant ainsi le biseau de l'aiguille 2 qui pourra ainsi traverser la membrane 5 du dispositif et libérer le sang vers le corps de la seringue 9 prévu à cet effet.

Une fois la seringue remplie l'utilisateur d'un geste ferme désolidarise l'ensemble seringue dispositif 1, 2, 3, 4, 5, 6, 7 assurant l'intégrité et l'étanchéité du prélèvement sanguin destiné au laboratoire.

*Fig : 1*

EP 0 457 631 A1

L'invention concerne un dispositif permettant de transférer du sang artériel ou veineux dans une seringue, sans risque de contamination par le sang ni par piqûres pour le préleveur ; de plus la technique utilisée avec ce dispositif permet d'effectuer en une seule ponction sur le patient, et sans mélange d'air, aussi bien le prélèvement destiné aux gaz du sang que le prélèvement destiné aux examens de laboratoire.

Cette technique préserve le capital artériel ou veineux du patient.

Traditionnellement, le prélèvement du sang artériel destiné à l'examen des gaz du sang s'effectue à l'aide d'une seringue héparinée et d'une aiguille ; compte tenu de la pression artérielle, il n'est pas possible de laisser l'aiguille en place le temps d'échanger la seringue à gaz du sang avec une autre seringue destinée à d'autres analyses, sans risque de contamination par le sang pour le préleveur.

Une autre technique consiste à prélever du sang par l'intermédiaire d'un vacutainer, surtout lorsque plusieurs prélèvements sont nécessaires pour réaliser d'une part, les examens de laboratoire divers, d'autre part, l'analyse des gaz du sang. La difficulté dans ce dernier cas étant de faire coincider le Luer mâle de la seringue avec l'aiguille intérieure du vacutainer rendant le prélèvement de sang délicat, voire impossible.

Le dispositif selon l'invention permet de remédier à ces inconvénients. Il comporte en première caractéristique un corps cylindrique en forme de piston évidé en son milieu de part en part, recevant à l'une de ses extrémités un Luer Lock femelle obstrué par une membrane en latex munie d'un cône directeur prolongé par un capillaire destiné à rentrer dans le cône Luer mâle de la seringue qui sera vissé ultérieurement, ce qui permet d'éviter tout espace mort favorisant la formation de bulles d'air dans le sang ainsi recueilli ; l'air étant proscrit à toutes analyses des gaz du sang. A l'autre extrémité du corps cylindrique se trouve une butée destinée à limiter ultérieurement l'introduction de l'aiguille du vacutainer vers le cône Luer mâle de la seringue.

Le dispositif ainsi vissé est introduit dans le vacutainer. La partie cylindrique, en forme de piston, du dispositif permet de centrer parfaitement la membrane en latex en face de l'aiguille intérieure du vacutainer.

Sous une simple pression du piston ou corps cylindrique du dispositif sur l'aiguille du vacutainer, l'utilisateur, d'un seul geste, perfore la protection en latex de l'aiguille du vacutainer et la membrane en latex du dispositif, permettant ainsi l'introduction de l'aiguille dans la partie Luer mâle de la seringue, limitée dans sa pénétration juste à l'entrée du capillaire, par la butée du corps cylindrique du dispositif.

Une fois la seringue remplie, l'utilisateur d'un geste ferme désolidarise l'ensemble seringue-dispositif, assurant l'intégrité et l'étanchéité du prélèvement sanguin destiné au laboratoire.

Les dessins annexés illustrent l'invention.

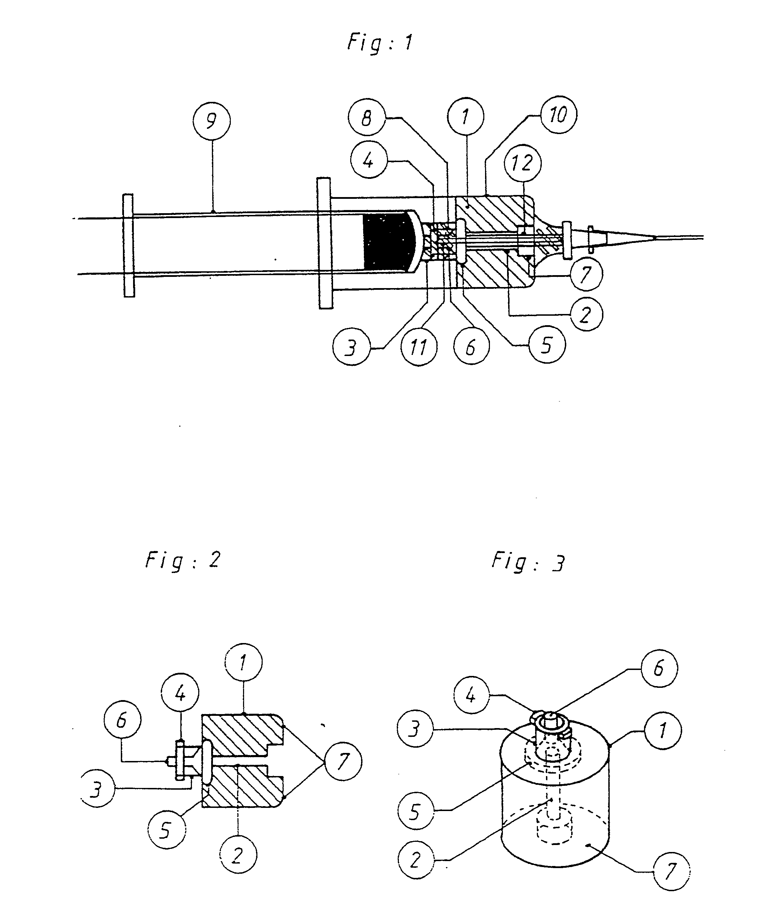

La figure 1 représente en coupe le dispositif selon l'invention.

La figure 2 représente en perspective le dispositif selon l'invention.

La figure 3 représente le fonctionnement du dispositif selon l'invention.

En référence à ces dessins, le dispositif comporte un corps cylindrique (1) en forme de piston, évidé en son milieu de part en part (2), recevant à l'une de ses extrémités un Luer femelle (3) Lock (4) obstrué par une membrane en latex (5), munie d'un cône directeur (6) prolongé par un capillaire destiné à rentrer dans le cône Luer mâle (8) de la seringue (9) qui sera vissé ultérieurement. A l'autre extrémité du corps cylindrique (1) se trouve une butée (7). L'extrémité du Luer Lock femelle (3) et (4) est dans un premier temps vissée sur le Luer Lock mâle (8) du corps de la seringue (9).

Le dispositif ainsi vissé est introduit dans le vacutainer (10).

La partie cylindrique en forme de piston (1) du dispositif, permet de centrer parfaitement la membrane en latex (5) en face de l'aiguille intérieure (11) du vacutainer (10).

Sous une simple pression du piston ou corps cylindrique (1) du dispositif sur l'aiguille (11) du vacutainer (10), l'utilisateur d'un seul geste perfore la protection en latex (12) de l'aiguille 11 du vacutainer (10) et la membrane en latex (5) du dispositif, permettant ainsi l'introduction de l'aiguille (11) dans la partie Luer Lock mâle (8) de la seringue, limitée dans sa pénétration juste à l'entrée du capillaire par la butée (7) du corps cylindrique (1).

Le corps cylindrique (1, 2, 7) sera réalisé en matière soit nylon, soit PVC, soit polypropilène, soit polysulphone, et tous autres dérivés de ces matières en une seule opération de moulage par injection. Le corps cylindrique (1, 2, 7) pourra aussi être réalisé en silicone, latex, caoutchouc, et tous dérivés de ces matières, par moulage.

La membrane (5) sera réalisée en matière, latex, caoutchouc, silicone, et autres dérivés de ces matières, par moulage.

Le Luer Lock femelle (3, 4) et le cône directeur muni de son capillaire (6) seront réalisés en matière polycarbonate, nylon, PVC, polysulphone, polypropilène et tous autres dérivés de ces matières en une seule opération de moulage par injection.

A titre d'exemple non limitatif, le diamètre du corps cylindrique sera de l'ordre de 18 mm et la hauteur de l'ordre de 20 mm.

Le dispositif selon l'invention est particulièrement destiné au prélèvement du sang permettant l'analyse des gaz du sang.

## Revendications

**(1)** Dispositif permettant le transfert du sang artériel ou veineux d'un système vacutainer à une seringue, caractérisé en ce qu'il comporte un corps cylindrique (1) en forme de piston évidé en son milieu de part en part (2) recevant à l'une de ses extrémités une membrane en latex (5) munie d'un cône directeur (6). A l'autre extrémité du corps cylindrique se trouve une butée (7). Le dispositif ainsi vissé sur la seringue (9), grâce à son extrémité munie d'un Luer Lock femelle (3,4) est introduit dans le vacutainer permettant de centrer parfaitement la membrane en latex (5) en face de l'aiguille du vacutainer (11), la simple pression du piston ou corps cylindrique du dispositif sur l'aiguille (11) du vacutainer (10) permettant à l'utilisateur, d'un seul geste, de perforer la protection en latex (12) de l'aiguille (11) du vacutainer (10) et la membrane en latex (5) du dispositif, permettant ainsi l'introduction de l'aiguille (11) dans le cône directeur (6) du dispositif, limitée dans sa pénétration, juste à l'entrée du capillaire, par la butée (7) du corps cylindrique (1).

**(2)** Dispositf selon la revendication (1), caractérisée en ce que le corps cylindrique (1) constitue une partie du dispositif destinée à centrer parfaitement l'aiguille intérieure (11) du vacutainer (10) en face le Luer Lock mâle (8) du corps de la seringue (9).

**(3)** Dispositif selon la revendication (2) caractérisée en ce que la membrane en latex va repousser, par simple pression, la protection de l'aiguille (12) intérieure du vacutainer libérant ainsi l'aiguille (11) qui à son tour perforera la membrane en latex (5) du dispositif permettant ainsi l'écoulement du sang dans la seringue (9).

**(4)** Dispositif selon la revendication (3) caractérisée en ce que le cône (6) directeur muni de son capillaire (6) va permettre de diriger l'aiguille (12) au centre du capillaire lui même réduisant l'ouverture du cône Luer mâle de la seringue (9) évitant ainsi tout espace mort pouvant favoriser la formation de bulles d'aire dans le prélèvement sanguin.

**(5)** Dispositif selon la revendication (4) caractérisée en ce que la butée (7) limite la course de l'aiguille (11) intérieure du vacutainer juste à l'entrée du capillaire (6).

**(6)** Dispositif selon la revendication (5) caractérisée en ce que chaque partie du dispositif (1, 2, 3, 4, 5, 6, 7) sera réalisée en nylon, PVC, polypropilène, polysulphone, latex, silicone, caoutchouc, ou tout autre dérivé de ces matières, par moulage ou par injection.

Fig : 1

Fig : 2

Fig : 3

Office européen
des brevets

**RAPPORT DE RECHERCHE EUROPEENNE**

Numero de la demande

EP 91 40 0943

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | GB-A-1 036 000 (S. & R. J. EVERETT & CO. LTD.) <br> * page 2, lignes 43-81,103-107; figures 1-4 * | 1-6 | A 61 B 5/14 |
| A | US-A-3 736 932 (F.E. SATCHELL) <br> * colonne 5, ligne 49 - colonne 6, ligne 38; figure 7 * | 1-5 | |
| A | EP-A-0 055 859 (WALTER SARSTEDT KUNSTSTOFF-SPRITZGUSSWERK) <br> * page 4, ligne 15 - page 6, ligne 31; figures 1-3 * | 1-5 | |

| | DOMAINES TECHNIQUES RECHERCHES (Int. Cl.5) |
|---|---|
| | A 61 B 5/00 <br> A 61 M 5/00 |

Le présent rapport a été établi pour toutes les revendications

| Lieu de la recherche | Date d'achèvement de la recherche | Examinateur |
|---|---|---|
| BERLIN | 15-07-1991 | WEIHS J.A. |